# EUROPÄISCHE PATENTANMELDUNG

(11) **EP 4 759 333 A1**
(43) Veröffentlichungstag der Anmeldung: **17.06.2026**
(21) Anmeldenummer: 24220378.4
(22) Anmeldetag: 16.12.2024
(51) Int. Cl.: A61L 2/26, B65B 3/00, B65B 35/36

(54) **VERARBEITUNGSVORRICHTUNG FÜR OBJEKTE IN EINER KONTROLLIERTEN UMGEBUNG**

(71) Anmelder: TT Innovation AG, 6343 Rotkreuz (CH)
(72) Erfinder: ZELLER, Mike, 4246 Wahlen (CH); MÜLLER, Mathieu, 68510 Sierentz (FR)
(74) Vertreter: Mertzlufft-Paufler, Cornelius

(57) **Zusammenfassung**

Es wird eine Verarbeitungsvorrichtung (1) für Objekte (2) und ein Verfahren zur Verarbeitung von Objekten (2) in einer kontrollierten Umgebung (3) vorgeschlagen, wobei die kontrollierte Umgebung (3) wenigstens eine Passage (4) zum Einbringen zumindest von Objekten (2), die in Gebinden (5) in einem ersten Format (6), das wenigstens eine flächige Anordnung bildet und/oder wenigstens eine Anordnung von n x m Objekten (2), wobei n > 1 und m > 1, umfasst, vorliegen, aufweist, wobei die Gebinde (5) in einer Verpackung (7) bereitgestellt werden, die zumindest teilweise außerhalb der kontrollierten Umgebung (3) verbleibt, dadurch gekennzeichnet, dass in der kontrollierten Umgebung (3) eine Umsetzvorrichtung (8) zu einem Umsetzen (9) der Objekte (2) von dem ersten Format (6) n x m auf ein zweites Format (10) o x p, wobei p = 1 und o ≥ 1, ausgebildet ist.

## Beschreibung

Die Erfindung betrifft eine Verarbeitungsvorrichtung für Objekte in einer kontrollierten Umgebung, wobei die kontrollierte Umgebung wenigstens eine Passage zum Einbringen zumindest von Objekten, die in Gebinden in einem ersten Format vorliegen, aufweist, wobei die Gebinde in einer Verpackung bereitgestellt werden, die zumindest teilweise außerhalb der kontrollierten Umgebung verbleibt.

Beispielsweise kann das Format eine flächige Anordnung bilden. In diesem Fall ist die Anordnung nicht durch eine Gerade beschreibbar. Ein Beispiel kann eine spiralförmig gewickelte oder eine radiale Anordnung oder eine Anordnung mit konzentrischen Kreisen sein. Es kann auch vorgesehen sein, dass das Format eine Anordnung von n x m Objekten, wobei n > 1 und m > 1, umfassen kann. Hierbei können zusätzlich Randbereiche mit weiteren Behältern aufgefüllt sein.

Die Erfindung betrifft weiter ein Verfahren zur Verarbeitung von Objekten, insbesondere Objekten in einer kontrollierten Umgebung, wobei die Objekte als ein Gebinde in einem ersten Format n x m, wobei n > 1 und m > 1, durch eine Passage der kontrollierten Umgebung zugeführt werden.

Die Erfindung betrifft weiter ein Verfahren zur Verarbeitung von Objekten in einer kontrollierten Umgebung, wobei eine Eingangs-Transportvorrichtung und/oder eine Umsetzvorrichtung und/oder eine Zwischen-Umsetzvorrichtung und/oder eine Ausgangs-Umsetzvorrichtung die Objekte mit einem Greifinstrument greift und/oder umsetzt.

Aufgabe der Erfindung ist es derartige Vorrichtungen zu verbessern. Aufgabe der Erfindung ist es auch derartige Verfahren zu verbessern.

Zur Lösung der genannten Aufgabe sind erfindungsgemäß die Merkmale des Anspruchs 1 vorgesehen. Insbesondere wird somit zur Lösung der genannten Aufgabe bei einer Verarbeitungsvorrichtung der eingangs beschriebenen Art erfindungsgemäß vorgeschlagen, dass in der kontrollierten Umgebung eine Umsetzvorrichtung zu einem Umsetzen der Objekte von dem ersten Format n x m auf ein zweites Format o x p, wobei p = 1 und o ≥ 1, ausgebildet ist. Hierdurch kann beispielsweise erreichbar sein, dass die Objekte im zweiten Format in der kontrollierten Umgebung weiterverarbeitet werden können, wobei das zweite Format für verschiedene Bearbeitungsprozesse von Vorteil ist. So ist beispielsweise ein Erreichen einer Objektöffnung, anfahrend rechtwinklig zu p möglich, ohne über eine Objektöffnung zu greifen, da p = 1 ist. Das zweite Format o x p kann p = 1 und o > 1 sein. Das zweite Format o x p kann auch p = 1 und o = 1 sein. Das Format der Objekte kann als zweidimensionale Anordnung definiert sein, wobei n entlang einer Achse der Anordnung verläuft und m entlang einer anderen Achse der Anordnung verläuft, wobei die Achsen n und m quer zueinander verlaufen. Die Zahl definiert die Anzahl der Objekte, die entlang dieses Formats angeordnet sind. Das Format o kann beispielsweise entlang einer Transportrichtung definierbar sein. Das Format o x p kann sich auf eine jeweilige Transporteinheit beziehen, wobei o innerhalb eines gesamten Prozesses in der kontrollierten Umgebung geteilt und/oder verkleinert werden kann. Das Format o x p muss nicht zwangsläufig entlang einer Linie verlaufen, sondern kann auch Abweichung von der Linie beinhalten, beispielsweise eine zick-zack-Linie, wobei die Objekte leicht zueinander versetzt angeordnet sind. Jedes Objekt einer Anordnung kann einen eindeutigen Vorgänger und Nachfolger haben, abgesehen von den Objekten die an einem Anfang und Ende einer Anordnung angeordnet sind. Die Verpackung kann ein sogenanntes Tub sein, das eine Aufnahme für medizinische und/oder pharmazeutische Objekte bildet. Die Verpackung kann dabei eine Abdeckung, beispielsweise eine Abdeckfolie, aus einem Vliesgewebe, wie beispielsweise Tyvek, aufweisen. Die Verpackung kann im Innern eine Objektaufnahme, ein sogenanntes Nest, aufweisen, dass die Objekte aufnehmen kann. Die Verpackung kann eine Umverpackung aufweisen, wobei die Umverpackung, vor einem Transfer von Objekten aus der Verpackung in die kontrollierte Umgebung, entfernt werden kann. Das Gebinde kann die Objektaufnahme und die Objekte umfassen. Durch das Gebinde können die vereinzelten Objekte zu einer einfacher handhabbaren Einheit werden. Die Verpackung, worin ein Gebinde gelagert ist, kann, bei dem Transfer von Objekten in die kontrollierte Umgebung, von außerhalb der kontrollierten Umgebung an eine Passage der kontrollierten Umgebung gehalten werden. Eine Handlingsvorrichtung kann die Abdeckung der Verpackung öffnen und eine Objektabdeckung, die die Objekte abdeckt, entfernen. Insbesondere kann vorgesehen sein, dass ein Objekt ein, vorzugsweise unbefüllter oder befüllter, Behälter, ein Verschlusselement, beispielsweise ein Stopfen oder ein Plunger, oder ein Sicherungselement, beispielsweise eine Kappe, oder ein Verbrauchsmaterial, beispielsweise ein Beutel, oder ein anderes Teil, das in einem pharmazeutischen Abfüllprozess und/oder Fertigungsprozess verwendbar ist. Es kann vorgesehen sein, dass die Passage eine Schleuse zum Einschleusen von Objekten in die kontrollierte Umgebung ist.

Die n x m Objekte eines Gebindes können beispielsweise einzeln, in Gruppen oder als Ganzes durch die Passage gebracht werden.

Bei dem Transfer kann die Verpackung außerhalb der kontrollierten Umgebung verbleiben. Hierdurch kann beispielsweise erreichbar sein, dass potenzielle Kontaminationen der kontrollierten Umgebung unterbleiben. Ein Einschleusen der Verpackung könnte hingegen zu einer potenziellen Kontamination der kontrollierten Umgebung führen.

Ebenfalls kann es vorteilhaft sein, die Verpackung nicht in die kontrollierte Umgebung einzuschleusen, da die Verpackung außerhalb der kontrollierten Umgebung an Prozessstationen vorbeigeführt werden kann, so dass die Verpackung am Ende der kontrollierten Umgebung wieder zur Befüllung mit behandelten Objekten zur Verfügung steht. Je weniger Objekte in der kontrollierten Umgebung vorliegen, umso weniger Transportvorrichtungen werden benötigt, was Kosten und Komplexität reduziert. Ein Teil der Verpackung, beispielsweise die Objektaufnahme und/oder die Objektabdeckung können in die kontrollierte Umgebung transferiert werden. Es ist auch denkbar, dass die Objekte aus der Objektaufnahme in die kontrollierte Umgebung transferiert werden und die Objektaufnahme in der Verpackung verbleibt und nicht in die kontrollierte Umgebung verbracht wird.

Eine kontrollierte Umgebung kann beispielsweise als ein begrenzter Raumbereich charakterisierbar sein, in welchem definierte Umgebungsbedingungen, insbesondere in Bezug auf eine Luftreinheit und/oder eine Oberflächenreinheit, schaffbar und/oder aufrechterhaltbar sind, beispielsweise durch eine Kontrolle eines Luftaustausches mit einer Außenwelt der kontrollierten Umgebung. Die Kontrolle des Luftaustausches kann beispielsweise durch einen völligen Ausschluss des Luftaustausches im Normalbetrieb (beispielsweise bis auf eine geschlossene Luftzirkulation) oder eine konsequente Vorgabe einer Richtung des Luftaustausches (in die kontrollierte Umgebung oder aus der kontrollierten Umgebung) realisiert sein. Beispiele kontrollierter Umgebungen umfassen Isolatoren, Restricted Access Barrier Systems (zugangsbeschränkte Barrierensysteme, insbesondere vom offenen oder geschlossenen Typ), Containments, Gloveboxes. Innerhalb der kontrollierten Umgebung, insbesondere des Containments oder des Isolators, können somit verschiedene Prozesse zur Verarbeitung verschiedenster Erzeugnisse, beispielsweise pharmazeutischer Erzeugnisse, erfolgen, wobei die Gefahr einer Kontamination wenigstens eines Erzeugnisses durch Partikel und/oder Verunreinigungen von außerhalb der kontrollierten Umgebung dezimiert werden kann. Solche pharmazeutischen Erzeugnisse können beispielsweise jeweils ein pharmazeutisches Objekt zu einer Aufnahme eines pharmazeutischen Präparats aufweisen. Eine Liste von Beispielen solcher pharmazeutischen Objekte umfasst zumindest Vials, Spritzen, Karpulen (Zylinderampullen) und sonstige befüllbare pharmazeutische Behälter oder auch medizinische Abgabevorrichtungen insbesondere welche die Objekte enthalten. Weiter können solche Objekte auch andere im Umfeld des Fill Finish Prozesses benötigte Mittel / Hilfsmittel sein, insbesondere Verschlussmittel wie Stopfen oder Sicherungsmittel wie Kappen. Die kontrollierte Umgebung kann insbesondere für die Behandlung und/oder Verarbeitung von pharmazeutischen Erzeugnissen vorgesehen sein, beispielsweise zur Befüllung und/oder Verschließung von Objekten, insbesondere von Behältern, mit pharmazeutischen Produkten. Die kontrollierte Umgebung kann Filter aufweisen, die eine Luft filtern, bevor diese in die kontrollierte Umgebung eintritt. Die Filter befinden sich beispielsweise oberhalb der Objekte, die in der kontrollierten Umgebung behandelt werden.

Die Verpackung der Objekte kann die Passage, von außen verschließen. Außen kann dabei als eine Umgebung außerhalb der kontrollierten Umgebung definiert sein. Außen kann dabei beispielsweise eine weniger kontrollierte Umgebung als die kontrollierte Umgebung sein. Außen kann aber beispielsweise auch eine zweite kontrollierte Umgebung sein.

Die Umsetzvorrichtung kann beispielsweise eine Handlingsvorrichtung sein, die Objekte, die im ersten Format n x m vorliegen, aufnehmen kann und in einem zweiten Format o x p absetzen kann. Die Objekte können dabei von der Umsetzvorrichtung im zweiten Format aufgenommen werden. Dabei können die Objekte im zweiten Format beispielsweise auf magnetisch levitierbaren Movern abgesetzt werden. Es kann insbesondere vorgesehen sein, dass die Objekte auch im ersten Format auf Movern und/oder einem anderen Transportsystem bereitgestellt werden.

Bei einer vorteilhaften Ausgestaltung kann vorgesehen sein, dass zwischen der Passage und der Umsetzvorrichtung eine Eingangs-Transportvorrichtung ausgebildet ist, die zum Transport zumindest der Objekte im ersten Format n x m eingerichtet ist. Die Eingangs-Transportvorrichtung kann beispielsweise eine Handlingsvorrichtung sein, die die Objekte, die in der Verpackung im ersten Format n x m vorliegen, im ersten Format n x m transportiert. Die Eingangs-Transportvorrichtung kann die Objekte beispielsweise in dem ersten Format n x m greifen und aus der Verpackung, insbesondere an der Objektaufnahme gegriffen, entnehmen. Hierdurch können beispielsweise vorteilhaft nur die Objekte und/oder die Objektaufnahme entnommen werden, und die Verpackung oder zumindest Teile davon nicht eingebracht werden, was die Wahrscheinlichkeit einer Kontamination der kontrollierten Umgebung verringert. Auch kann so beispielsweise darauf verzichtet werden die Objektaufnahme in der kontrollierten Umgebung zu verarbeiten. Insbesondere kann vorgesehen sein, dass die Eingangs-Transportvorrichtung zum Transport der Gebinde im ersten Format n x m zwischen der Passage und der Umsetzvorrichtung eingerichtet ist. Die Eingangs-Transportvorrichtung kann beispielsweise auch die Objektaufnahme greifen und diese mit samt der Objekte aus der Verpackung entnehmen. Somit können beispielsweise alle Objekte mit der Objektaufnahme in einem Arbeitsschritt von der Eingangs-Transportvorrichtung gegriffen werden. Dies kann die Bearbeitungszeit in der kontrollierten Umgebung verkürzen.

Bei einer vorteilhaften Ausgestaltung kann vorgesehen sein, dass die Verpackung zu einem Abdichten der Passage eingerichtet ist. Hierdurch kann beispielsweise sichergestellt werden, dass kein Luftaustausch zwischen der kontrollierten Umgebung und außerhalb der kontrollierten Umgebung stattfinden kann, wenn die Verpackung an der Passage abdichtend gehalten ist. Alternativ kann ein Teil der Verpackung zu einem Abdichten der Passage eingerichtet sein. Dieser Teil kann die Abdeckung sein. Hierdurch kann beispielsweise die Passage gegen die Abdeckung der Verpackung abdichten. Abdichten kann ebenfalls bedeuten, dass die Verpackung so an der Passage gehalten ist, dass ein Spalt zwischen der Verpackung und der kontrollierten Umgebung entsteht, durch den Luft aus der kontrollierten Umgebung fließen kann. So kann auch ohne einen direkten Kontakt zwischen der Verpackung und der kontrollierten Umgebung ein Eindringen von Verschmutzungen verhindert werden.

Bei einer vorteilhaften Ausgestaltung kann vorgesehen sein, dass ein Teil der Verpackung durch die Passage transferierbar ist. Ein Teil der Verpackung kann beispielsweise ein zuvor in der Passage dekontaminiertes Teil sein, dass die Objekte schützt und zur Entnahme der Objekte entfernt werden muss. Um den Arbeitsfluss zu gewährleisten und Zugriff auf die darunterliegenden Objekte, insbesondere Behälter zu erhalten, kann der Teil der Verpackung durch die Passage in die kontrollierte Umgebung transferierbar sein. Vorzugsweise ist eine Abdeckfolie der Verpackung durch die Passage transferierbar. Die Abdeckfolie kann zuvor dekontaminiert worden sein und nach dem Transfer durch die Passage einen Zugang zu den Objekten freigeben. Nach dem Entfernen wird eine solche Abdeckfolie vorzugsweise in der kontrollierten Umgebung gelagert und/oder aus dieser entfernt, insbesondere durch eine Schleuse oder eine "lufttechnisch abgeschirmte Öffnung". Ein Teil der Verpackung kann auch die Objektabdeckung sein.

Bei einer vorteilhaften Ausgestaltung kann vorgesehen sein, dass eine Zwischenstation eingerichtet ist, auf der die Objekte im ersten Format n x m verarbeitet und/oder transportiert werden. Die Zwischenstation kann zwischen der Eingangs-Transportvorrichtung und der Umsetzvorrichtung ausgebildet sein. Hierdurch kann beispielsweise erreichbar sein, dass ein Bearbeitungsschritt der Objekte im ersten Format n x m an der Zwischenstation erfolgen kann. Insbesondere kann vorgesehen sein, dass die Objekte auf der Zwischenstation abgesetzt werden. Hierdurch kann beispielsweise die Zwischenstation als Puffer-Station dienen, so dass die Eingangs-Transportvorrichtung weitere Verpackungen bearbeiten kann, während die Umsetzvorrichtung noch mit einem anderen Arbeitsschritt beschäftigt ist. Vorzugsweise sind die Objekte noch als Gebinde angeordnet. Dies kann bedeuten, dass die Objekte in der Objektaufnahme angeordnet sind. Somit können vorteilhaft alle Objekte, als Gebinde, zusammen transportiert oder verarbeitet werden. Die Zwischenstation kann als levitierbarer Mover ausgebildet oder auf einem levitierbaren Mover angeordnet sein. Die Objekte können von der Eingangstransportvorrichtung auf die Zwischenstation abgesetzt werden. Dabei kann es von Vorteil sein, dass die Zwischenstation einen Aufsatz aufweist, wobei der Aufsatz die Objekte als Gebinde aufnehmen kann. Wenn die Objekte als Behälter in einem Gebinde vorliegen, kann der Aufsatz mit der Objektaufnahme so zusammenwirken, dass die Behälter relativ zur Objektaufnahme angehoben werden, so dass die Behälter einfach von der Umsetzvorrichtung gegriffen werden können. Der levitierbare Mover kann die Zwischenstation gegenüber der Umsetzvorrichtung beispielsweise so positionieren, dass der Mover die Objekte für ein Greifen der Umsetzvorrichtung anordnen kann, so dass die Umsetzvorrichtung stets die gleiche Bewegung ausführen kann, ohne ihr Greifinstrument nach den Objekten ausrichten zu müssen.

Bei einer vorteilhaften Ausgestaltung kann vorgesehen sein, dass die Zwischenstation beweglich ist. Hierdurch kann beispielsweise eine Zusammenarbeit von Eingangs-Transportvorrichtung und Zwischenstation und/oder Eingangs-Transportvorrichtung und Umsetzvorrichtung vereinfacht sein, da die Zwischenstation sich beispielsweise zur Eingangs-Transportvorrichtung und/oder Umsetzvorrichtung hin- und wegbewegen kann. Insbesondere kann vorgesehen sein, dass die Zwischenstation drehbar ist. Hierdurch kann beispielsweise ein von der Eingangs-Transportvorrichtung auf der Zwischenstation abgestelltes Gebinde zur Umsetzvorrichtung hingedreht werden. Alternativ oder zusätzlich kann vorgesehen sein, dass die Zwischenstation verfahrbar ist. Hierdurch kann beispielsweise eine zusätzliche Flexibilität und Beweglichkeit der Zwischenstation erreichbar sein. Alternativ oder zusätzlich kann vorgesehen sein, dass die Zwischenstation die Objekte für die Umsetzvorrichtung präsentiert. Hierdurch kann beispielsweise erreichbar sein, dass die Umsetzvorrichtung die Objekte vorteilhaft einfach entgegennehmen kann. Die Zwischenstation und/oder die Eingangs-Transportvorrichtung kann beispielsweise die Orientierung des Gebindes relativ zur Umsetzvorrichtung ändern, so dass Zeilen und Spalten vertauscht sind. Das Format n x m kann so zum Format m x n werden. Die Zwischenstation und/oder die Eingangs-Transportvorrichtung kann dazu eingerichtet sein, die Gebinde so zu orientieren, dass die Objekte durch die Umsetzvorrichtung gegriffen werden können.

Bei einer vorteilhaften Ausgestaltung kann vorgesehen sein, dass wenn m kein Teiler, wobei m eine natürliche Zahl > 0 sein muss, oder kein Vielfaches von p ist, eine Orientierung des Formats der Objekte durch die Eingangs-Transportvorrichtung und/oder Zwischenstation so veränderbar ist, dass der Umsetzvorrichtung n präsentiert wird. Hierdurch kann beispielsweise erreichbar sein, dass die Umsetzvorrichtung die Objekte aufnehmen kann.

Bei einer vorteilhaften Ausgestaltung kann vorgesehen sein, dass zumindest ein Mover die Zwischenstation so bewegt, dass die Umsetzvorrichtung bei einer Greifbewegung die Objekte an einem selben Ort wie bei einer vorherigen Greifbewegung greift. Hierdurch kann beispielsweise erreichbar sein, dass die Umsetzvorrichtung einen Greifer immer an die gleiche Stelle bewegen kann, so dass keine Feinjustierungen notwendig sind. Alternativ oder zusätzlich kann vorgesehen sein, dass ein Mover die Zwischenstation bewegt. Hierdurch kann beispielsweise die Zwischenstation eine zusätzliche Beweglichkeit und Flexibilität erlangen. Alternativ oder zusätzlich kann vorgesehen sein, dass die Umsetzvorrichtung bei einer Absetzbewegung die Objekte an einem selben Ort wie bei einer vorherigen Absetzbewegung absetzt. Hierdurch kann insbesondere erreichbar sein, dass die Umsetzvorrichtung die Objekte schneller absetzen kann, da keine Feinausrichtung bei der Absetzung nötig ist. Insbesondere kann vorgesehen sein, dass ein Mover die zu greifenden Objekte positioniert. Hierdurch kann beispielsweise erreichbar sein, dass die zu greifenden Objekte einfacher gegriffen werden können. Alternativ oder zusätzlich kann vorgesehen sein, dass ein Mover Absetzpositionen positioniert. Hierdurch kann beispielsweise erreichbar sein, dass ein Absetzen von Objekten schneller möglich sein kann, wenn eine Absetzposition so bereitgestellt ist, dass die Umsetzvorrichtung eine vorteilhaft kurze Bewegung durchführen kann.

Bei einer vorteilhaften Ausgestaltung kann vorgesehen sein, dass die Verarbeitungsvorrichtung wenigstens eine Bearbeitungsstation aufweist, wobei der Bearbeitungsstation vor- und/oder nachgeordnet wenigstens eine Zwischen-Transportvorrichtung eingerichtet ist. Hierdurch kann beispielsweise erreichbar sein, dass die Bearbeitungsstation Objekte behandeln kann und dass die Objekte mit der Zwischen-Transportvorrichtung an die oder von der Bearbeitungsstation bewegt werden können. Insbesondere kann vorgesehen sein, dass die Zwischen-Transportvorrichtung zu einem Transport der Objekte im zweiten Format o x p eingerichtet ist. Hierdurch kann beispielsweise die Zwischen-Transportvorrichtung die Objekte im zweiten Format hin- und von der Bearbeitungsstation wegbewegen. Insbesondere kann vorgesehen sein, dass eine Dimension o in Transportrichtung der Zwischen-Transportvorrichtung ausgerichtet ist. Die Zwischen-Transportvorrichtung kann die Objekte beispielsweise in einer Reihe anordnen, wenn p = 1 und o > 1. Die auf der Zwischen-Transportvorrichtung aufgereihten Objekte können so beispielsweise einfach zu einer Bearbeitungsstation in der kontrollierten Umgebung bewegt werden und dort bearbeitet werden. Bearbeitungsstationen können beispielsweise Befüllstationen oder Verschließstationen sein. Vor- und/oder nachgeordnet, kann sich auf einen Materialfluss innerhalb der kontrollierten Umgebung beziehen.

Bei einer vorteilhaften Ausgestaltung kann vorgesehen sein, dass der Abstand der Objekte zueinander im Format o x p veränderlich ist. Hierdurch kann beispielsweise eine flexible Anordnung der Objekte in Bezug auf die Schnittstellen zu Transport oder Bearbeitungsstationen gegebenen Bedingungen erreicht werden. Beispielsweise kann eine Bearbeitungsstation einen bestimmten Abstand der Objekte zueinander vorschreiben. Insbesondere kann vorgesehen sein, dass der Abstand beim Transport der Objekte mit der Zwischen-Transportvorrichtung oder vor einer Bearbeitung der Objekte an einer Bearbeitungsstation verändert oder beibehalten wird. Somit können die Objekte beispielsweise vorteilhaft schnell bearbeitet werden und ein Transport oder eine Pufferung kann platzsparend durchgeführt werden.

Bei einer vorteilhaften Ausgestaltung kann vorgesehen sein, dass die Verarbeitungsvorrichtung eine Zwischen-Umsetzvorrichtung aufweist, die die Objekte von einem Format o x p zu o x p' umsetzt. Hierdurch kann beispielsweise erreichbar sein, dass ein bestimmter Abstand zwischen den Behältern eingestellt werden kann, insbesondere bevor diese den Bearbeitungsstation(en) zugeführt werden, welcher von vorzugsweise allen oder zumindest einer nachfolgenden Bearbeitungsstation als Schnittstelle verwendet wird.

Die Zwischen-Umsetzvorrichtung kann in Bezug auf angetriebene Bewegungsachsen analog zu der Umsetzvorrichtung ausgebildet sein, insbesondere zu einer Aufnahme der Objekte von der Zwischen-Transportvorrichtung.

Allgemein kann die Zwischen-Transportvorrichtung zu einer Bewegung quer zum Materialfluss eingerichtet sein, um eine Ausrichtung der Objekte zu einer vorangehenden und/oder nachfolgenden Umsetzvorrichtung zu erleichtern.

Bei einer vorteilhaften Ausgestaltung kann vorgesehen sein, dass die Zwischen-Umsetzvorrichtung in einer selben Zone wie die Umsetzvorrichtung angeordnet ist. Die Zone kann hierbei beispielsweise eine begrenzte Umgebung gleicher Reinheit, insbesondere gleichen oder unterschiedlichen Drucks sein. Hierdurch kann beispielsweise erreichbar sein, dass zwischen der Zwischen-Umsetzvorrichtung und der Umsetzvorrichtung keine oder für das Produkt positiv geänderten Umgebungsbedingungen vorherrschen. Beispielsweise können die Zwischen-Umsetzvorrichtung und die Umsetzvorrichtung im selben Isolator angeordnet sein. Dies kann ein effizientes Zusammenarbeiten der beiden Vorrichtungen ermöglichen. Alternativ kann vorgesehen sein, dass die Zwischen-Umsetzvorrichtung in der selben Zone wie eine Ausgangs-Umsetzvorrichtung angeordnet ist. Hierdurch kann beispielsweise ein effizientes Zusammenarbeiten der Ausgangs-Umsetzvorrichtung und der Zwischen-Umsetzvorrichtung ermöglicht sein.

Bei einer vorteilhaften Ausgestaltung kann vorgesehen sein, dass die Verarbeitungsvorrichtung eine, beispielsweise die bereits erwähnte, Ausgangs-Umsetzvorrichtung aufweist, die die Objekte vom o x p' in ein Format n' x m` umsetzt, wobei n' > 1 und m' > 1 ist. Hierdurch kann die Ausgangs-Umsetzvorrichtung die Objekte in ein Format umsetzen, dass für die Weiterbearbeitung der Objekte, in einem Materialfluss nach der Ausgangs-Umsetzvorrichtung, gewünscht ist. P' kann dabei gleich p sein oder ein Teiler oder ein Vielfaches von p sein. Insbesondere kann vorgesehen sein, dass n' = n ist. Alternativ oder zusätzlich kann vorgesehen sein, dass m` = m ist. Hierdurch kann beispielsweise erreichbar sein, dass das Format in das die Objekte durch die Ausgangs-Umsetzvorrichtung umgesetzt werden, das gleiche ist, wie das Format, in dem die Objekte in der Verpackung für die Bearbeitung in der kontrollierten Umgebung angeliefert wurden. Insbesondere kann vorgesehen sein, dass die Ausgangs-Umsetzvorrichtung die Objekte im Format n' x m` in eine Objektaufnahme, beispielsweise die bereits erwähnte Objektaufnahme oder in eine Verpackung, beispielsweise die bereits erwähnte Verpackung, umsetzt. Alternativ kann vorgesehen sein, dass die Objekte in eine andere Anordnung gebracht werden. Die verarbeiteten Objekte können beispielsweise in ein Einschubbehälter eingeschoben werden, der eine große Anzahl von Objekten aufnehmen kann.

Bei einer vorteilhaften Ausgestaltung kann vorgesehen sein, dass eine Objektaufnahme-Transportvorrichtung separat von der Zwischen-Transportvorrichtung ausgebildet ist. Hierdurch kann beispielsweise erreichbar sein, dass die Objektaufnahme-Transportvorrichtung die Objektaufnahme transportieren kann, während die Zwischen-Transportvorrichtung die Objekte transportieren kann. Alternativ oder zusätzlich kann vorgesehen sein, dass eine, beispielsweise die bereits erwähnte, Objektaufnahme separat von der Umsetzvorrichtung ausgebildet ist. Hierdurch kann beispielsweise erreichbar sein, dass die Umsetzvorrichtung die Objekte umsetzen kann, während die Objektaufnahme-Transportvorrichtung die Objektaufnahme transportieren kann. Insbesondere kann vorgesehen sein, dass die Objektaufnahme-Transportvorrichtung von einem Materialfluss abzweigt. Hierdurch kann beispielsweise erreichbar sein, dass die Objektaufnahme-Transportvorrichtung die Objektaufnahme separat von einem Materialfluss der Objekte transportiert. Alternativ oder zusätzlich kann die Objektaufnahme-Transportvorrichtung in einen, beispielsweise den bereits erwähnten, Materialfluss der Objekte münden. Hierdurch kann beispielsweise erreichbar sein, dass die Objektaufnahme an einem Ende des Materialflusses für eine Befüllung mit Objekten wieder zur Verfügung steht.

Bei einer vorteilhaften Ausgestaltung kann vorgesehen sein, dass die Objektaufnahme-Transportvorrichtung zumindest teilweise außerhalb der kontrollierten Umgebung ausgebildet ist. Hierdurch kann beispielsweise erreichbar sein, dass die Objektaufnahme aus der kontrollierten Umgebung herausgebracht wird, um diese nicht einer unnötigen potenziellen Kontamination auszusetzen. Dies kann bei toxischen Anwendungen von Vorteil sein. Es kann auch vorgesehen sein, dass die kontrollierte Umgebung nicht durch eine potenziell kontaminierte Objektaufnahme kontaminiert wird. Dies kann beispielsweise bei aseptischen Anwendungen wichtig sein.

Bei einer vorteilhaften Ausgestaltung kann vorgesehen sein, dass die Ausgangs-Umsetzvorrichtung und die Umsetzvorrichtung in unterschiedlichen Reinheitszonen angeordnet sind. Hierdurch kann beispielsweise erreichbar sein, dass die Ausgangs-Umsetzvorrichtung in einer Zone geringerer Reinheit angeordnet ist, beispielsweise in einem RABS, in der die verschlossenen Objekte in die Objektaufnahme gesetzt werden. Eine solche Zone geringerer Reinheit ist kostengünstiger umzusetzen und zu betreiben, als beispielsweise ein Isolator.

Bei einer vorteilhaften Ausgestaltung kann vorgesehen sein, dass die Objektaufnahme in die kontrollierte Umgebung eingebracht wird. Hierdurch kann beispielsweise erreichbar sein, dass in der Objektaufnahme vorliegende Objekte als ein Gebinde umgesetzt werden können. Alternativ oder zusätzlich kann vorgesehen sein, dass die Verarbeitungsvorrichtung eine Schleuse aufweist, um die Objektaufnahme nach einer Objektentnahme aus der kontrollierten Umgebung zu schleusen. Hierdurch kann die Objektaufnahme beispielsweise einer Objektaufnahme-Transportvorrichtung, die sich außerhalb der kontrollierten Umgebung befindet, zugeführt werden. Die Schleuse kann Mittel zur Dekontamination und/oder Sterilisation umfassen und Objekte beispielsweise mit H2O2, UVC-Strahlung und/oder E-Beam bearbeiten. Die Schleuse kann einen Druckunterschied zu einer Umgebung außerhalb der Schleuse aufweisen, wobei der Druckunterschied beispielsweise eine Kontamination der kontrollierten Umgebung verhindern kann.

Bei einer vorteilhaften Ausgestaltung kann vorgesehen sein, dass die Bearbeitungsstation zu einem gleichzeitigen Verarbeiten von o Objekten eingerichtet ist. Hierdurch kann beispielsweise erreichbar sein, dass alle o Objekte die von einer Zwischen-Transportvorrichtung die o Objekte tragen kann, gleichzeitig an einer Bearbeitungsstation verarbeitet werden können. Dies kann eine Bearbeitungszeit an der Bearbeitungsstation verkürzen. Das Verarbeiten kann insbesondere ein Befüllen und/oder Verschließen und/oder Sichern und/oder Inspizieren und/oder Beschriften und/oder Analysieren der Objekte oder deren Inhalt oder Verschlussmittel oder Abgabemittel sein. Alternativ oder zusätzlich kann die Bearbeitungsstation zur einem gleichzeitigen Verarbeiten von einem Bruchteil b von o Objekten eingerichtet sein. Hierdurch können auch Teilmengen von o Objekten verarbeitet werden. Alternativ oder zusätzlich kann vorgesehen sein, dass die Bearbeitungsstation zu einem gleichzeitigen Verarbeiten von einem Vielfachen von o Objekten eingerichtet ist. Somit kann beispielsweise eine volle Auslastung der Bearbeitungsstation sichergestellt werden, was die Bearbeitungszeit in kontrollierten Umgebung vorteilhaft verkürzen kann. Vorzugsweise kann b ein Teiler als natürliche Zahl von o sein. Somit kann beispielsweise eine Auslastung der Bearbeitungsstation vorteilhaft gesteuert werden. Alternativ oder zusätzlich kann vorgesehen sein, dass b= ½*o, 1/3*o, ¼*o ist. Beispielsweise kann eine Bearbeitungsstation so viele Bearbeitungsplätze aufweisen, wie Objekte vorgesehen sind, womit eine Auslastung der Bearbeitungsstation vorteilhaft gesteuert werden kann, insbesondere die Taktung einer Gesamtanlage.

Bei einer vorteilhaften Ausgestaltung kann vorgesehen sein, dass o ein Teiler von n*m ist. Ein Teiler kann als ganze oder natürliche Zahl a*o = n oder a*o = m definiert sein. Hierdurch kann beispielsweise erreichbar sein, dass das erste Format n x m ohne Rest in das Format o x p umgesetzt werden kann.

Bei einer vorteilhaften Ausgestaltung kann vorgesehen sein, dass die Zwischenstation zumindest einen magnetisch levitierbaren Mover hat. Mover sind beispielsweise von Planarantrieben bekannt. Hierdurch kann beispielsweise erreichbar sein, dass die Objekte auf dem Mover abgesetzt werden und mit dem Mover zur Umsetzvorrichtung transportiert werden können. Alternativ oder zusätzlich kann vorgesehen sein, dass die Zwischen-Transportvorrichtung zumindest einen levitierbaren Mover hat. Mit derartigen Movern kann eine große Flexibilität im Transport der Objekte in der kontrollierten Umgebung geschaffen werden. Die Zwischen-Transportvorrichtung kann beispielsweise mit auf ihr angeordneten Objekten eine Befüllstation anfahren und im Anschluss eine Verschließstation. Somit kann auf ein Umladen der Objekte verzichtet werden.

Bei einer vorteilhaften Ausgestaltung kann vorgesehen sein, dass die Umsetzvorrichtung höchstens zwei Bewegungsachsen, insbesondere nur eine Bewegungsachse hat. Dies ermöglicht schnelle Bewegungsabläufe, wodurch eine hohe Taktrate in der gesamten Produktionsstraße erreichbar ist.

Hierbei kann vorgesehen sein, dass eine, beispielsweise die bereits erwähnte, Eingangs-Transportvorrichtung und/oder eine, beispielsweise die bereits erwähnte, Zwischenstation zu einer Positionierung der Objekte relativ zu der Umsetzvorrichtung eingerichtet ist. Dies kann eine Verarbeitung des einkommenden Formats selbst bei einer reduzierten Zahl von verfügbaren Bewegungsachsen an der Umsetzvorrichtung erleichtern. Eine Ausrichtung der zu greifenden Objekte kann somit erfolgen, während die Umsetzvorrichtung vorangehenden Objekte umsetzt.

Zur Lösung der eingangs genannten Aufgabe sind alternativ oder zusätzlich die Merkmale des auf ein Verfahren gerichteten Anspruchs vorgesehen. Insbesondere wird somit ein Verfahren zur Verarbeitung von Objekten in einer kontrollierten Umgebung, vorgeschlagen, wobei die Objekte in einer Verpackung bereitgestellt werden, wobei die Objekte in einem ersten Format n x m, wobei n > 1 und m > 1, durch eine Passage der kontrollierten Umgebung zugeführt werden und in der kontrollierten Umgebung durch eine Umsetzvorrichtung in ein zweites Format o x p, wobei p = 1, o > 1, umgesetzt werden, wobei die Verpackung zumindest teilweise außerhalb der kontrollierten Umgebung verbleibt. Hierdurch kann beispielsweise erreichbar sein, dass die Objekte im zweiten Format einfacher von Bearbeitungsstationen in der kontrollierten Umgebung verarbeitet werden können, da die Bearbeitungsstationen beispielsweise auf eine Anlieferung der Objekte im zweiten Format angewiesen sind oder da die Bearbeitungsstationen die Objekte im zweiten Format schneller verarbeiten können. Zudem kann weniger Material in die kontrollierte Umgebung eingebracht werden, wodurch das Risiko einer potenziellen Kontamination verringert werden kann. Durch das Einbringen von weniger Material wird weniger Platz in der kontrollierten Umgebung benötigt und die Verarbeitung kann weniger komplex sein, da weniger Einzelteile verarbeitet und in einen Prozessfluss integriert werden müssen. Insbesondere kann vorgesehen sein, dass die Objekte als ein Gebinde vorliegen. Hierdurch kann beispielsweise erreichbar sein, dass die Objekte einfacher handhabbar sein können.

Bei einer bevorzugten Anwendung kann vorgesehen sein, dass eine Eingangs-Transportvorrichtung, die zwischen der Passage und der Umsetzvorrichtung ausgebildet ist, ein Gebinde transportiert. Die Eingangs-Transportvorrichtung kann beispielsweise die Gebinde zur Umsetzvorrichtung transportieren, wo diese in das zweite Format umgesetzt werden können. Die Eingangs-Transportvorrichtung kann beispielsweise auch die Gebinde aus der an der Passage gehaltenen Verpackung entnehmen und weitertransportieren, insbesondere kann diese die Verpackung öffnen.

Bei einer vorteilhaften Anwendung kann vorgesehen sein, dass die Eingangs-Transportvorrichtung die Objekte in dem Format n x m auf einer Zwischenstation absetzt. Hierdurch kann beispielsweise erreichbar sein, dass die Eingangs-Transportvorrichtung und die Umsetzvorrichtung unabhängiger voneinander arbeiten können, da die Zwischenstation einen Puffer bildet und die Objekte aufnehmen kann. Alternativ oder zusätzlich kann vorgesehen sein, dass eine Zwischenstation, beispielsweise die bereits erwähnte Zwischenstation, die Objekte der Umsetzvorrichtung präsentiert. Hierdurch kann beispielsweise erreichbar sein, dass die Zwischenstation die Objekte so für die Umsetzvorrichtung anbieten kann, dass die Umsetzvorrichtung die Objekte vorteilhaft einfach aufnehmen kann.

Bei einer vorteilhaften Anwendung kann vorgesehen sein, dass die Objekte nach der Umsetzvorrichtung im zweiten Format o x p transportiert werden. Nach der Umsetzvorrichtung kann sich auf einen Materialfluss in der kontrollierten Umgebung beziehen. Hierdurch können die Objekte beispielsweise der Bearbeitungsvorrichtung in der kontrollierten Umgebung im zweiten Format zugeführt werden, was für die Verarbeitung der Objekte vorteilhaft sein kann.

Bei einer vorteilhaften Anwendung kann vorgesehen sein, dass nach der Umsetzung ein Abstand der Objekte zueinander verändert wird. Die Abstandsänderung kann beispielsweise durch die Zwischen-Transportvorrichtung erfolgen. Hierdurch kann der Abstand der Objekte beispielsweise an die Bearbeitungsstation angepasst werden.

Bei einer vorteilhaften Anwendung kann vorgesehen sein, dass eine Objektaufnahme in die kontrollierte Umgebung eingebracht wird. Hierdurch kann beispielsweise erreichbar sein, dass eine Vielzahl von Objekten, die in der Objektaufnahme angeordnet sind, in einem Arbeitsschritt in die kontrollierte Umgebung transferiert werden können. Dadurch wird der Inhalt der Verpackung in einem kurzen Arbeitsschritt entnommen, wodurch unmittelbar im Anschluss der Prozess zur Entfernung der Verpackung eingeleitet werden kann, oder wodurch unmittelbar im Anschluss die Verpackung mit einem anderen, vorzugsweise auszuschleusendem, Objekt befüllt werden kann. Alternativ oder zusätzlich kann vorgesehen sein, dass eine Objektaufnahme, beispielsweise die bereits erwähnte Objektaufnahme, nach einer Entnahme der Objekte aus der Objektaufnahme, beispielsweise gemeinsam mit oder getrennt von der Verpackung, durch die Passage oder eine Schleuse nach außerhalb der kontrollierten Umgebung geschleust wird. Hierdurch kann eine potenzielle Kontamination der kontrollierten Umgebung durch die Objektaufnahme weniger wahrscheinlich werden. Die Objektaufnahme kann in einen Teil der Verpackung zurückgesetzt werden. Die Schleuse kann an einem Beginn des Materialflusses in der kontrollierten Umgebung angeordnet sein, sodass die Objektaufnahme an einem Beginn des Materialflusses aus der kontrollierten Umgebung geschleust werden kann. Sie kann aber auch "in der Mitte" eines Verarbeitungsprozesses angeordnet sein, um vorteilhaft zu verarbeitendes Material in die kontrollierte Umgebung einzubringen.

Bei einer vorteilhaften Anwendung kann vorgesehen sein, dass die Objektaufnahme zumindest teilweise außerhalb der kontrollierten Umgebung transportiert wird. Hierdurch kann beispielsweise eine Anzahl von Teilen in der kontrollierten Umgebung reduziert werden, was sich positiv auf die Reinheit der kontrollierten Umgebung auswirken kann. Auch kann beispielsweise erreichbar sein, dass eine Verarbeitung der Objekte nicht durch einen Transport der Objektaufnahme beeinflusst wird. Insbesondere kann vorgesehen sein, dass die Objektaufnahme mit den verarbeiteten Objekten bestückt wird. Hierdurch kann beispielsweise die Objektaufnahme wiederverwendet werden, was Kosten senken kann. Alternativ oder zusätzlich kann vorgesehen sein, dass ein Materialfluss der Objektaufnahmen von einem Materialfluss der Objekte abzweigt. Hierdurch kann beispielsweise erreichbar sein, dass die Objektaufnahme, nachdem die Objekte entnommen wurden, aus der kontrollierten Umgebung herausgeschleust wird. Alternativ oder zusätzlich kann vorgesehen sein, dass ein Materialfluss der Objektaufnahmen in einen Materialfluss der Objekte mündet. Hierdurch kann beispielsweise erreichbar sein, dass die Objektaufnahmen zur erneuten Bestückung mit Objekten, insbesondere Objekten und/oder Verschlussmitteln dem Materialfluss zugeführt werden kann. Die Bestückung der wiederverwendeten Objektaufnahmen mit den verarbeiteten Objekten kann in der kontrollierten Umgebung oder einer weniger kontrollierten Umgebung, beispielsweise einem RABS, erfolgen.

Bei einer vorteilhaften Anwendung kann vorgesehen sein, dass die umgesetzten Objekte befüllt werden. Hierdurch können die Objekte beispielsweise eine medizinische Substanz aufnehmen.

Alternativ oder zusätzlich kann vorgesehen sein, dass die Objekte verschlossen und/oder die Verschlussmittel gesichert werden. Hierdurch kann beispielsweise erreichbar sein, dass die Substanz im Objekt vor äußeren Einflüssen geschützt werden kann.

Bei einer vorteilhaften Anwendung kann vorgesehen sein, dass gleichzeitig o Objekte verarbeitet werden. Hierdurch kann ein Materialfluss in der kontrollierten Umgebung beschleunigt werden. Alternativ oder zusätzlich kann vorgesehen sein, dass gleichzeitig ein Bruchteil b von o Objekten verarbeitet wird. Hierdurch kann beispielsweise eine Bearbeitungsstation bedient werden, die einen Bruchteil b von o Objekten verarbeiten kann. Alternativ oder zusätzlich kann vorgesehen sein, dass gleichzeitig ein Vielfaches von o Objekten verarbeitet werden. Hierdurch kann beispielsweise die Verarbeitung von Objekten in der kontrollierten Umgebung beschleunigt werden. Insbesondere kann vorgesehen sein, dass die Objekte befüllt werden. Hierdurch können beispielsweise gleichzeitig o Objekte mit einer medizinischen Substanz befüllt werden. Alternativ oder zusätzlich kann vorgesehen sein, dass die Objekte verschlossen werden. Hierdurch kann beispielsweise erreichbar sein, dass gleichzeitig o Objekte verschlossen werden können. Vorzugsweise kann b ein Teiler von o sein. Hierdurch kann beispielsweise erreichbar sein, dass keine Objekte als Rest zurückbleiben. Alternativ oder zusätzlich kann vorgesehen sein, dass b= ½*o, 1/3*o, ¼*o ist. Hierdurch können beispielsweise Anpassungen an eine Verarbeitungsmöglichkeit von Bearbeitungsstationen möglich sein.

Zur Lösung der eingangs genannten Aufgabe sind alternativ oder zusätzlich die Merkmale des auf ein Verfahren gerichteten Anspruchs vorgesehen. Insbesondere wird somit ein Verfahren zur Verarbeitung von Objekten in einer kontrollierten Umgebung vorgeschlagen, wobei eine Eingangs-Transportvorrichtung die Objekte, insbesondere die Objektaufnahme und/oder Objekte mit einem Greifinstrument greift, wobei das Greifinstrument zu einer Aufnahme so geführt wird, dass ein potenzieller Transport von Verschmutzungen, vom Greifinstrument auf die Objekte geringgehalten wird. Hierdurch kann beispielsweise eine vorteilhafte Reinheit in der kontrollierten Umgebung erhalten werden. Alternativ oder zusätzlich kann vorgesehen sein, dass eine Umsetzvorrichtung und/oder eine Zwischen-Umsetzvorrichtung und/oder eine Ausgangs-Umsetzvorrichtung die Objekte mit einem Greifinstrument greift, wobei das Greifinstrument zu einer Aufnahme so geführt wird, dass ein potenzieller Transport von Verschmutzungen, vom Greifinstrument auf die Objekte geringgehalten wird. Unter dem Greifen und Führen, zum Geringhalten einer potenziellen Verschmutzung wird insbesondere verstanden, dass Luft, nach ihrem Austritt aus einem Endständigen (HEPA) Filter, keine andere Oberfläche trifft oder überstreicht als jene Oberflächen welche später direktem Produktkontakt ausgesetzt sind, insbesondere Oberflächen eines Objektes. Hierdurch kann beispielsweise vorteilhaft das Risiko einer Kontamination von bestimmten Bereichen in der kontrollierten Umgebung zusätzlich minimiert werden. Alternativ oder zusätzlich kann vorgesehen sein, dass eine Eingangs-Transportvorrichtung und/oder eine Umsetzvorrichtung und/oder eine Zwischen-Umsetzvorrichtung und/oder eine Ausgangs-Umsetzvorrichtung die Objekte mit einem Greifinstrument umsetzt, wobei das Greifinstrument zu einer Aufnahme so geführt wird, dass ein potenzieller Transport von Verschmutzungen, vom Greifinstrument auf die Objekte geringgehalten wird. Somit kann beispielsweise sichergestellt werden, dass das Greifinstrument oder ein anderes bewegliches oder starres Teil, welches nicht ein endständiger Filter ist, nicht über Objektöffnungen verfährt, wenn es sich vermeiden lässt. Alternativ oder zusätzlich kann vorgesehen sein, dass das Greifinstrument zu einem Absetzen so geführt wird, dass ein potenzieller Transport von Verschmutzungen, vom Greifinstrument auf die Objekte geringgehalten wird. Hierdurch kann beispielsweise erreichbar sein, dass beim Absetzten der Objekte das Greifinstrument nicht über offene Objektöffnungen verfährt. Insbesondere kann vorgesehen sein, dass ein potenzieller Transport on Verschmutzungen, in Bezug auf eine Luftströmung, auf die Objekte geringgehalten wird. Im Sinne der Erfindung können zum Greifinstrument auch weitere Ausleger oder andere bewegliche Teile gehören.

Bei einer bevorzugten Anwendung kann vorgesehen sein, dass die zu entnehmenden Objekte so ausgewählt werden, dass eine Wegstrecke, die das Greifinstrument über dem Gebinde zurücklegt, minimiert wird. Insbesondere kann vorgesehen sein, dass die Wegstrecke so gewählt wird, dass immer zuerst das dem Greifinstrument am nächsten stehende Objekt ausgewählt wird, sodass das Greifinstrument und/oder ein bewegtes Teil, andere offene Objekte (Behälter) nicht übergriffen werden. Hierdurch kann beispielsweise eine Wahrscheinlichkeit einer Kontamination der Objekte durch das Greifinstrument oder ein anderes Bewegliches Teil, wie beispielsweise der Ausleger, an welchem das Greifinstrument befestigt ist, verringert werden. Alternativ oder zusätzlich kann vorgesehen sein, dass die zu entnehmenden Objekte so ausgewählt werden, dass ein Überschattungsbereich über dem Gebinde minimiert wird. Hierdurch kann beispielsweise erreichbar sein, dass das Greifinstrument keine unnötigen Wege über dem Gebinde zurücklegt, die die Wahrscheinlichkeit für eine Kontamination erhöhen würden. Alternativ oder zusätzlich kann die Wegstrecke und/oder der Überschattungsbereich, die/den das Greifinstrument zurücklegt über der Zwischenstation und/oder Zwischen-Transportvorrichtung minimiert werden. Hierdurch kann beispielsweise eine Kontamination der Zwischenstation und/oder der Zwischen-Transportvorrichtung verhindert oder vermindert werden. Alternativ oder zusätzlich kann vorgesehen sein, dass die Wegstrecke und/oder ein Überschattungsbereich, die das Greifinstrument zurücklegt, vermieden wird. Hierdurch kann beispielsweise am sichersten eine Kontamination verhindert werden.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen:
- Fig. 1: eine Seitenansicht eines Teils einer Verarbeitungsvorrichtung in einer kontrollierten Umgebung,
- Fig. 2: eine Seitenansicht eines Teils einer Verarbeitungsvorrichtung mit zwei Handlingsvorrichtungen,
- Fig. 3: eine Draufsicht auf eine Verarbeitungsvorrichtung,
- Fig. 4: eine seitliche Schnittdarstellung einer Verpackung,
- Fig. 5: eine seitliche Schnittdarstellung der Verpackung aus Fig. 4 in vereinfachter Darstellung
- Fig. 6: eine seitliche Schnittdarstellung der Verpackung aus Fig. 5 mit geöffneter Abdeckung und aufliegender Objektabdeckung,
- Fig. 7: eine Draufsicht auf ein Gebinde von Objekten,
- Fig. 8: eine weitere Draufsicht auf ein Gebinde von Objekten und
- Fig. 9: eine weitere Draufsicht auf das Gebinde von Objekten aus Fig. 8.

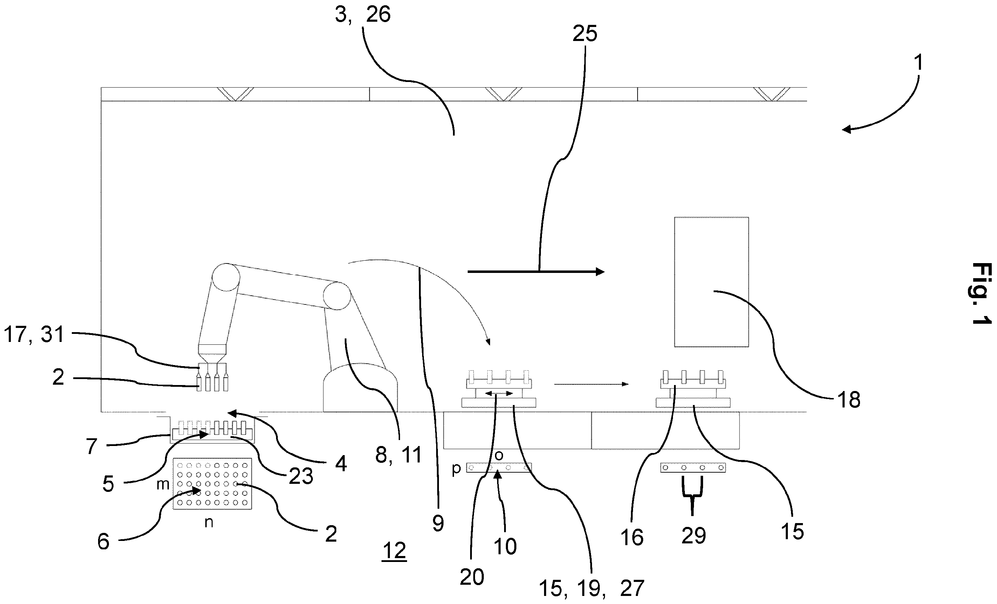

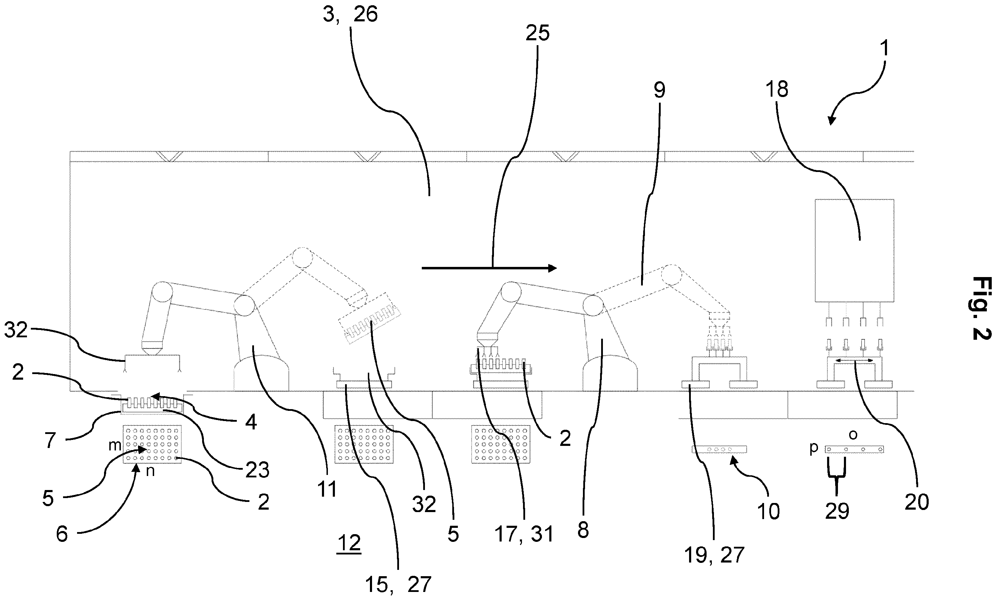

Fig. 1 zeigt eine Verarbeitungsvorrichtung 1 für Objekte 2 in einer kontrollierten Umgebung 3. Die kontrollierte Umgebung 3 weist eine Passage 4 zum Einbringen von Objekten 2 auf. Die Objekte 2 liegen in Gebinden 5 in einem ersten Format 6 n x m vor, wobei n = 8 und m = 5. Die Gebinde 5 sind in einer Verpackung 7 bereitgestellt. Die Verpackung 7 verbleibt teilweise außerhalb der kontrollierten Umgebung 3, während die Objekte 2 in die kontrollierte Umgebung 3 transferiert werden. In der kontrollierten Umgebung 3 ist eine Umsetzvorrichtung 8 angeordnet, die zu einem Umsetzen 9 der Objekte 2 von dem ersten Format 6, hier 8 x 5, auf ein zweites Format 10 o x p, wobei p = 1 und o = 4, ausgebildet ist. Die in Fig. 1 und 2 gezeigte kontrollierte Umgebung 3 ist auf der rechten Bildseite offen gezeichnet, um zu verdeutlichen, dass die kontrollierte Umgebung 3 weitere Stationen umfassen kann, die aus Platzgründen hier nicht gezeigt sind. Es ist selbstverständlich, dass die kontrollierte Umgebung 3 nicht ohne Weiteres in die Umgebung außerhalb der kontrollierten Umgebung 3 übergeht. Ein Übergang aus der kontrollierten Umgebung 3 nach außerhalb der kontrollierten Umgebung 3 kann beispielsweise durch geeignete Öffnungen, Ports, Mouseholes, Schnittstellen, Druckkonzepte, etc. erfolgen.

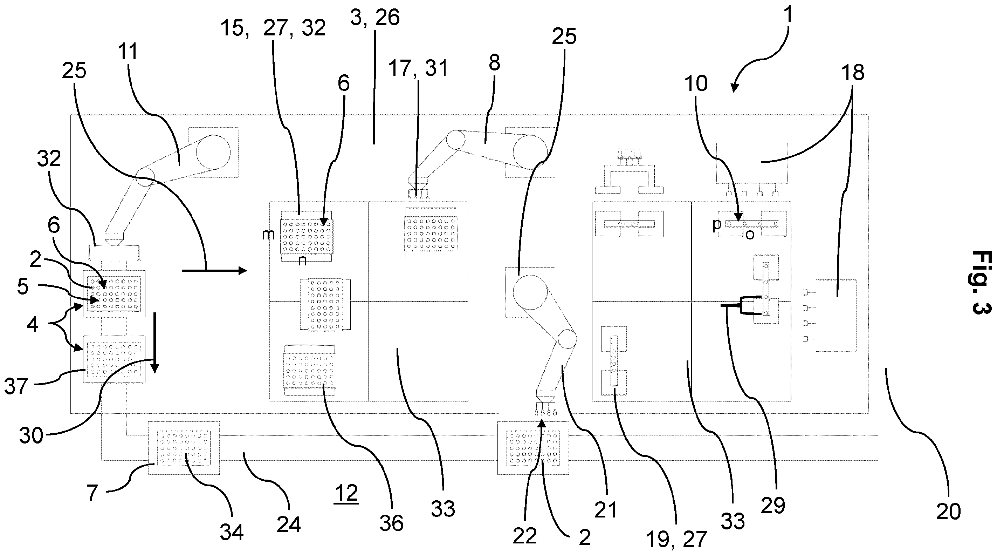

Die Passage 4 kann von innerhalb der kontrollierten Umgebung 3 mit einem Deckel 37, vorzugsweise mit einem integrierten Dekontaminationssystem, verschlossen werden. Fig. 3 offenbart eine kontrollierte Umgebung 3 mit zwei Passagen 4, wobei die Passagen 4 wechselweise mit dem Deckel 37 verschlossen werden können.

Die in Fig. 1 gezeigte Umsetzvorrichtung 8 entnimmt die Objekte 2 aus der Verpackung 7, die in der Verpackung 7 im ersten Format 6 vorliegen, im zweiten Format 10. Im Vergleich dazu entnimmt in Fig. 2 eine Eingangs-Transportvorrichtung 11 die Objekte 2 als Gebinde 5 im ersten Format 6 8 x 5 aus der Verpackung 7. Die Eingangs-Transportvorrichtung 11 kann die Objekte 2 als Gebinde 5 entnehmen. Alternativ kann die Eingangs-Transportvorrichtung 11 eine Objektaufnahme 23 entnehmen, die die Objekte 2 umfasst. Vorteilhaft kann die Eingangs-Transportvorrichtung 11 die Objektaufnahme 23 in die Verpackung 7 zurücksetzen.

Die Verpackung 7 dichtet gegen die Passage 4 ab, so dass kein Luftaustausch zwischen außen 12 und der kontrollierten Umgebung 3 stattfinden kann. Hierbei kann Außen 12 eine weitere kontrollierte Umgebung darstellen, insbesondere eine mit anderen regulatorischen Anforderungen.

Eine Abdeckung 13 und eine Objektabdeckung 14 der Verpackung 7 werden durch die Passage 4 in die kontrollierte Umgebung 3 transferiert (nicht gezeigt).

Fig. 2 zeigt, dass die Objekte 2 im ersten Format 6 8 x 5, durch die Eingangs-Transportvorrichtung 11 auf einer Zwischenstation 15 abgesetzt werden, wobei die Objekte 2 als Gebinde 5 vorliegen.

Die Zwischenstation 15 ist beweglich. Sie kann verfahren und sich drehen und die Objekte 2 für die Umsetzvorrichtung 8 präsentieren. Die Zwischenstation 15 ist dazu eingerichtet, das Gebinde 5 so zu orientieren, dass die Umsetzvorrichtung 8 eine Anzahl von Objekten 2 greifen kann, die für die Umsetzvorrichtung 8 vorteilhaft ist. In einem nicht gezeigten Ausführungsbeispiel kann die Zwischenstation auch eine Handlingseinheit, insbesondere ein Roboter oder durch einen Roboter angetrieben sein. In Fig. 1 greift die Umsetzvorrichtung 8 vier Objekte 2 aus einer Reihe von acht Objekten 2 und setzt die vier Objekte 2 auf die Zwischenstation 15 die vier Objektaufnahmen 16 aufweist. In dem in Fig. 3 gezeigten Ausführungsbeispiel ist sichtbar, dass die Zwischenstation 15 ihre Orientierung so ändern kann, dass die Umsetzvorrichtung 8 die Objekte 2 entsprechend ihrer Objektaufnahmen 17 greifen / aufnehmen kann. Die Orientierung der Objekte 2 wird von 5 x 8 auf 8 x 5 (und umgekehrt) geändert, indem sich die Zwischenstation 15 dreht. So kann die Umsetzvorrichtung 8 mit ihren vier Objektaufnahmen 17 vier Objekte 2 aus der Reihe von acht Objekten 2 greifen. In einem nachfolgenden Arbeitsschritt kann die Umsetzvorrichtung 8 dann die restlichen vier Objekte 2 aus der Reihe greifen.

Die Verarbeitungsvorrichtung 1 weist eine (Fig. 1 und 2) oder mehrere (Fig. 3) Bearbeitungsstationen 18 zur Verarbeitung der Objekte 2 auf. Der Bearbeitungsstation 18 vor- und nachgeordnet sind Zwischen-Transportvorrichtungen 19 angeordnet, die zu einem Transport der Objekte im zweiten Format 10, hier 4 x 1 eingerichtet sind. Eine Transportrichtung kann dabei als jene Richtung verstanden werden in der die Objekte 2 angeordnet. Die Transportrichtung entspricht hier der Erstreckung der vier Objekte 2.

Der Abstand 29 der Objekte 2 zueinander im zweiten Format 10 4 x 1 ist veränderlich. Eine Abstandsänderung 20 kann in einem Ruhezustand und/oder beim Transport der Objekte 2 mit der Zwischen-Transportvorrichtung 19 oder vor einer Bearbeitung in der Bearbeitungsstation 18 erfolgen. In einem nicht gezeigten Ausführungsbeispiel, kann der Abstand 29 auch durch die Umsetzvorrichtung, insbesondere durch einen verschiebbaren Greifer, bewerkstelligt werden.

In einem nicht gezeigten Ausführungsbeispiel weist die Verarbeitungsvorrichtung 1 eine Zwischen-Umsetzvorrichtung auf, die die Objekte von dem zweiten Format 10 o x p zu einem Format o x p' umsetzt.

Die Zwischen-Umsetzvorrichtung kann einer selben Zone wie die Umsetzvorrichtung 8 oder in einer selben Zone wie eine Ausgangs-Umsetzvorrichtung 21 angeordnet sein.

Die Verarbeitungsvorrichtung 1 umfasst die Ausgangs-Umsetzvorrichtung 21, die die Objekte von dem zweiten Format 10 o x p, hier 4 x 1, oder dem Format o x p' in ein Format n' x m` umsetzt. Das Umsetzen 22 der Ausgangs-Umsetzvorrichtung 21 erfolgt in eine Objektaufnahme 23 der Verpackung 7, wobei das Format n' > 1 und m' > 1 und n` = n und m' = m.

Die Verarbeitungsvorrichtung 1 weist eine Objektaufnahme-Transportvorrichtung 24 auf, die separat von der Zwischen-Transportvorrichtung 19 und separat von der Umsetzvorrichtung 8 ausgebildet ist. Die Objektaufnahme-Transportvorrichtung 24 zweigt von einem Materialfluss 25 der Objekte 2 ab und mündet wieder in den Materialfluss 25 der Objekte 2.

Die Objektaufnahme-Transportvorrichtung 24 ist außerhalb der kontrollierten Umgebung 3 ausgebildet. Die Objektaufnahme 23 wird aus der kontrollierten Umgebung 3 geschleust und außerhalb von der Objektaufnahme-Transportvorrichtung 24 aufgenommen.

Die Ausgangs-Umsetzvorrichtung 21 und die Umsetzvorrichtung 8 sind in einem nicht gezeigten Ausführungsbeispiel in unterschiedlichen Reinheitszonen 26 angeordnet. In dem Ausführungsbeispiel von Fig. 3 sind die Ausgangs-Umsetzvorrichtung 21 und die Umsetzvorrichtung 8 in einer Reinheitszone 26 angeordnet. Die Ausgangs-Umsetzvorrichtung kann die Objekte vorzugsweise so umsetzten, dass sie die eigene Zone nicht verlässt oder in die andere Zone eindringt. Dies, um vorteilhaft eine Kontaminationsübertragung zu vermeiden.

Die Objektaufnahme 23 der Verpackung 7 wird in die kontrollierte Umgebung 3 eingebracht und nach einer Objektentnahme der Objekte 2 durch eine Schleuse (nicht gezeigt) der Verarbeitungsvorrichtung 1 wieder aus der kontrollierten Umgebung 3 geschleust. Die Objektaufnahme 23 kann nach der Entnahme der Objekte 2 durch das Einlegen in eine offene, leere und an die Passage 4 angedockte Verpackung 7, insbesondere dieselbe oder eine andere, aus der kontrollierten Umgebung 3 ausgebracht werden. Dies kann zusammen oder ohne die vorgängig entfernte Abdeckung 13 und/oder Objekteabdeckung 14 geschehen.

In einer nicht gezeigten Ausführungsform kann die Objektaufnahme 23 in der kontrollierten Umgebung 3, parallel / separat zum eigentlichen Verarbeitungsprozess transportiert / mitgeführt werden, sodass am beispielsweise die Ausgangs-Umsetzvorrichtung die verarbeiteten Objekte 2 wieder in die Objektaufnahme 23 setzt.

In einer nicht gezeigten Ausführungsform kann die Objektaufnahme 23 durch eine Öffnung aus der kontrollierten Umgebung 3 ausgebracht werden, insbesondere durch einen Schlitz, welcher aussen mit vorbehandelter Luft überströmt wird, um einem Partikel und/oder mikrobiologischer Eintrag entgegen zu wirken.

Die Bearbeitungsstation 18 ist zu einer gleichzeitigen Verarbeitung von vier Objekten 2 oder einem Bruchteil b von vier Objekten 2 oder einer Vielzahl eingerichtet, wobei b = ½ oder b = ¼ ist.

Das Verarbeiten ist im vorliegenden Ausführungsbeispiel in Fig. 3 ein Befüllen und Verschließen der Objekte 2. Die Bearbeitungsstation 18 kann die Objekte 2 auch sichern, inspizieren, beschriften und/oder analysieren.

Die Zwischenstation 15 und die Zwischen-Transportvorrichtung 19 haben einen magnetisch levitierbaren Mover 27.

Die Objekte 2 werden als Gebinde 5 im ersten Format 6 8 x 5, durch die Passage 4 der kontrollierten Umgebung 3 zugeführt und in der kontrollierten Umgebung 3 mit einer Umsetzvorrichtung 8 in das zweite Format 10 4 x 1 umgesetzt, wobei die Verpackung 7 teilweise außerhalb der kontrollierten Umgebung verbleibt.

Die Eingangs-Transportvorrichtung 11, die zwischen der Passage 4 und der Umsetzvorrichtung 8 angeordnet ist, transportiert die Gebinde 5. Sie kann die Verpackung 7, durch entfernen, insbesondere Aufschneiden, der Abdeckung 13, auch öffnen und die Objekteabdeckung entnehmen (nicht gezeigt). Alternativ kann das Öffnen der Verpackung 7 und Freilegen der Objekte 2 / Objekte durch eine weitere nicht gezeigte Handlingseinheit vorgenommen werden.

Die Eingangs-Transportvorrichtung 11 kann als Mehrachs-Handlingseinheit ausgebildet sein, um komplexere Bewegungsabläufe realisieren zu können.

Die Umsetzvorrichtung 8 ist als 1-Achs-Handlingseinheit ausgebildet und realisiert daher nur einen Bewegungsgrad (hier eine gebogene Linie, die durch ein entsprechendes Getriebe und/oder geometrisch realisiert ist.) Die Zwischenstation 15, also hier der Mover 27, kann hierbei so verfahren werden, dass die zu greifenden Objekte 2 jeweils relativ zu der Umsetzvorrichtung 8 ausgerichtet sind.

Wird dieses Prinzip der relativen Ausrichtung auch bei der Eingangs-Transportvorrichtung 11 realisiert, lassen sich hier ebenfalls Bewegungsachsen einsparen. Fig. 2 und 3 zeigen, wie die Eingangs-Transportvorrichtung 11 die Objekte 2 im Format 8 x 5 auf der Zwischenstation 15 absetzt, wobei die Zwischenstation 15 die Objekte 2 der Umsetzvorrichtung 8 präsentiert.

Bezogen auf den Materialfluss 25, werden die Objekte 2 nach der Umsetzvorrichtung 8 im zweiten Format 10, also 4 x 1 transportiert.

Nach dem Umsetzung 9 wird der Abstand 29 der Objekte 2 zueinander verändert.

Die Objektaufnahme 23 wird in die kontrollierte Umgebung 3 eingebracht und nach der Entnahme der Objekte 2 aus der Objektaufnahme 23 durch die Passage 4 oder die Schleuse (nicht gezeigt) nach außerhalb der kontrollierten Umgebung 3 geschleust.

Fig. 3 zeigt, dass die Objektaufnahme 23 zeitweise innerhalb und zeitweise außerhalb der kontrollierten Umgebung 3 transportiert wird. Außerhalb der kontrollierten Umgebung kann die Objektaufnahme zur Ausgangs-Umsetzvorrichtung transportiert werden und dort mit den bearbeiteten Objekten bestückt werden. Wenn die Objektaufnahme 23 außerhalb transportiert wird, kann sie von der Ausgangs-Umsetzvorrichtung 21 mit den bearbeiteten Objekten 2 bestückt werden. Ein Materialfluss 30 der Objektaufnahme 23 zweigt von dem Materialfluss 25 der Objekte 2 ab und mündet später wieder in den Materialfluss 25 der Objekte 2.

Fig. 3 zeigt in der Draufsicht zwei Passagen 4, wobei eine Passage 4 gerade von der Eingangs-Transportvorrichtung 11 bearbeitet wird und die andere Passage 4 von innen mit dem Deckel 37 verschlossen ist.

Die umgesetzten Objekte 2 werden befüllt und verschlossen.

An einer Bearbeitungsstation 18 die vier Objekte 2 verarbeiten kann, können die vier Objekte gleichzeitig verarbeitet, also befüllt und/oder verschlossen, werden. Es können aber auch nur drei, zwei, oder ein Objekte 2 an der Bearbeitungsstation 18 verarbeitet werden. Ebenso ist denkbar, dass die Verarbeitungsstation mehr als vier, insbesondere acht Objekte gleichzeitig verarbeiten kann.

Die Eingangs-Transportvorrichtung 11, die Umsetzvorrichtung 8, die Zwischen-Umsetzvorrichtung und/oder die Ausgangs-Umsetzvorrichtung 21 greifen und/oder setzen die Objekte 2 um, wobei ein Greifinstrument 31 zu einem Aufnehmen und Absetzen der Objekte 2 so geführt wird, dass ein potenzieller Transport von Verschmutzungen auf die Objekte 2 geringgehalten wird.

Auch die Zwischen-Umsetzvorrichtung und/oder die Ausgangs-Umsetzvorrichtung 21 kann/können als Handlingseinheit mit höchstens zwei Bewegungsachsen, insbesondere höchstens einer Bewegungsachse ausgebildet sein, wie zuvor bei der Umsetzvorrichtung 8 beschrieben.

Die zu entnehmenden Objekte 2 werden so ausgewählt, dass eine Wegstrecke und ein Überschattungsbereich, die das Greifinstrument 31 über dem Gebinde 5 und/oder der Zwischenstation 15 und/oder der Zwischen-Transportvorrichtung zurücklegt, minimiert wird.

Die Eingangs-Transportvorrichtung 11 die in Fig. 2 gezeigt ist, greift die Objektaufnahme mit den darin als Gebinde vorliegenden Objekten.

Fig. 3 zeigt wie die Mover 27 sich frei auf einer Arbeitsfläche 33 bewegen können. Die Eingangs-Transportvorrichtung 11 legt die Gebinde 5 auf Zwischenstationen 15 ab. Die Zwischenstationen 15 können dann in der richtigen Orientierung die Objekte 2 der Umsetzvorrichtung 8 präsentieren. Nachdem alle Objekte 2 entnommen sind, kann die Zwischenstation 15 die entleerte Objektaufnahme 23 der Eingangs-Umsetzvorrichtung 11 präsentieren, so dass diese die entleerte Objektaufnahme 23 aus der Passage 4 nach außen 12 schleusen kann.

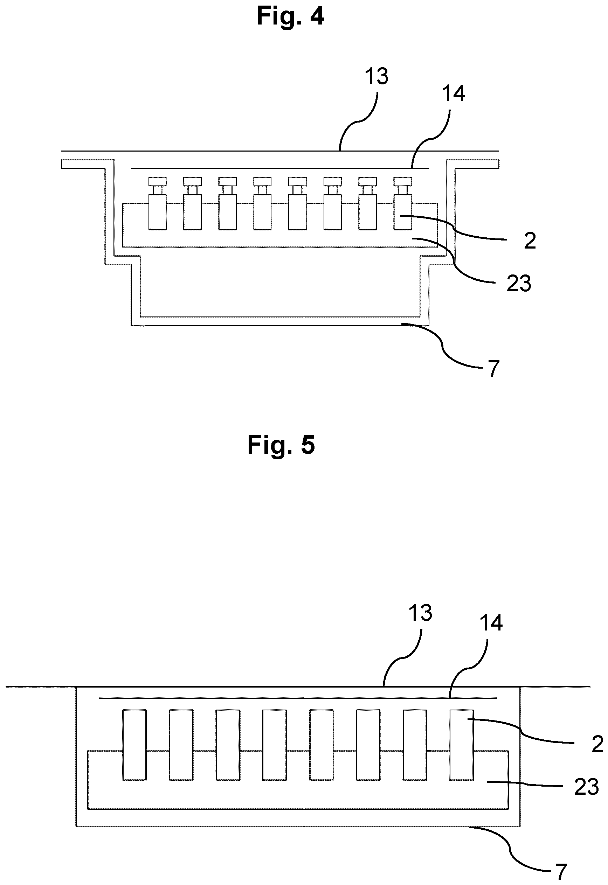

Die Fig. 4 - 6 zeigen Verpackungen 7 für Gebinde 5 von Objekten 2. Die Verpackung 7 weist eine Abdeckung 13 auf, die vorzugsweise aus dem Vliesstoff Tyvek besteht. Die Objekte 2 sind von einer Objekteabdeckung 14 abgedeckt. Der Innenraum 35 der Verpackung 7 ist aseptisch.

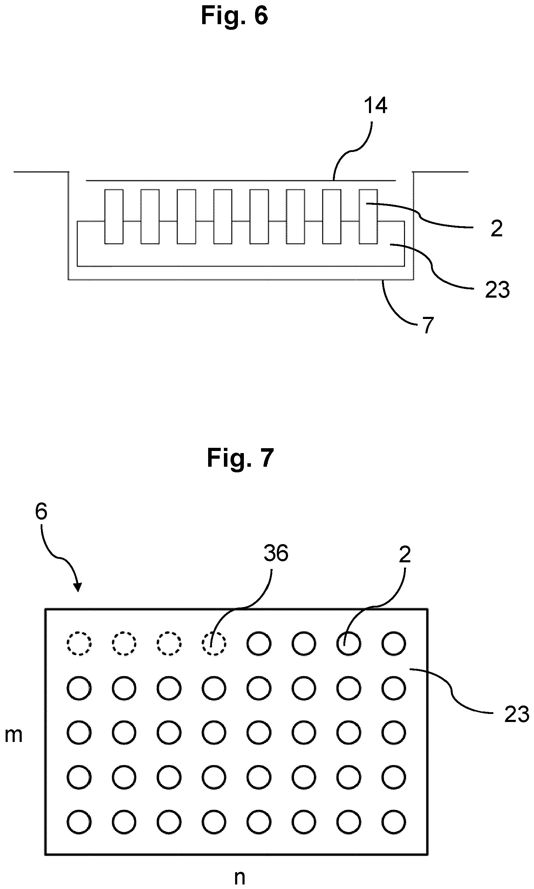

Fig. 7 zeigt eine Draufsicht auf ein teilentleertes Gebinde 5 von Objekten 2. Die Objekte 2 sind in der Objektaufnahme 23 angeordnet. Das gezeigte Gebinde 5 weist das erste Format 6 8 x 5 auf. Vier Objekte 2 wurden mit der Eingangs-Transportvorrichtung 11 oder der Umsetzvorrichtung 8 entnommen. Alternativ wurde die Behälteraufnahme bis auf vier leere Plätze, beispielsweise mit der Ausgangs-Transportvorrichtung, befüllt. Die leeren Plätze 36 der Objektaufnahme 23 sind gestrichelt gekennzeichnet.

Die in der Passage 4 angelieferten Objekte 2 werden in dem in Fig. 1 gezeigten Ausführungsbeispiel von der Umsetzvorrichtung 8 entnommen und umgesetzt. Eingangs-Transportvorrichtung 11 und Umsetzvorrichtung 8 sind diesem Ausführungsbeispiel dieselbe Vorrichtung. In dem in Fig. 2 und 3 gezeigten Ausführungsbeispiel sind die Eingangs-Transportvorrichtung 11 und die Umsetzvorrichtung 8 zwei getrennte Vorrichtungen. Dort entnimmt die Eingangs-Transportvorrichtung 11 die Gebinde 5. Die Umsetzvorrichtung 8 entnimmt dann die Objekte 2 von der Zwischenstation 15.

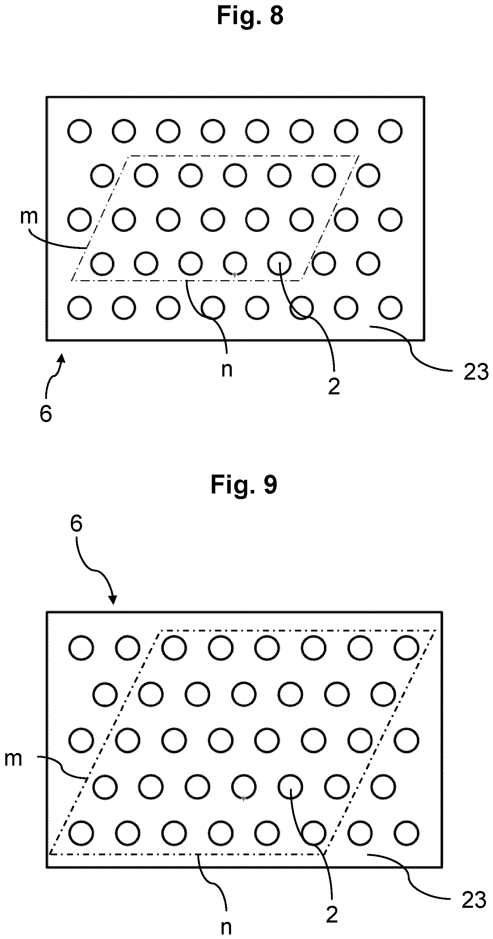

Fig. 8 zeigt ein Gebinde 5 des ersten Formats 6, wobei n = 5 und m = 3. Die gestrichpunktete Linie umrandet die Anordnung der Objekte 2 des Formats 6.

Fig. 9 zeig das Gebinde 5 aus Fig. 8, wobei das erste Format hier, n = 6 und m = 5 ist. Die Anordnung der Objekte im ersten Format n x m muss nicht der Entnahmereihenfolge der Objekte aus der Objektaufnahme entsprechen.

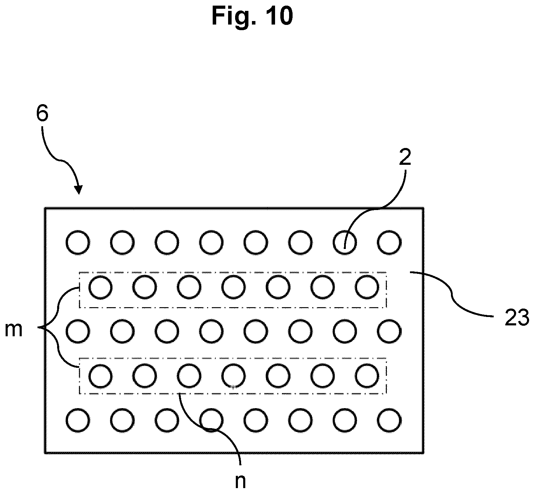

Fig. 10 zeigt das Gebinde aus Fig. 8 - 9, wobei das erste Format 6 hier, n = 7 und m = 2 ist. Das erste Format kann auch in n = 8 und m = 3 gesehen werden.

Das erste Format n x m kann folglich eine Teilmenge von Objekten aus einer Gesamtheit von Objekten in der Objektaufnahme sein.

Es wird eine Verarbeitungsvorrichtung 1 für Objekte 2 und ein Verfahren zur Verarbeitung von Objekten 2 in einer kontrollierten Umgebung 3 vorgeschlagen, wobei die kontrollierte Umgebung 3 wenigstens eine Passage 4 zum Einbringen zumindest von Objekten 2, die in Gebinden 5 in einem ersten Format 6, das wenigstens eine flächige Anordnung bildet und/oder wenigstens eine Anordnung von n x m Objekten (2), wobei n > 1 und m > 1, umfasst, vorliegen, aufweist, wobei die Gebinde 5 in einer Verpackung 7 bereitgestellt werden, die zumindest teilweise außerhalb der kontrollierten Umgebung 3 verbleibt, dadurch gekennzeichnet, dass in der kontrollierten Umgebung 3 eine Umsetzvorrichtung 8 zu einem Umsetzen 9 der Objekte 2 von dem ersten Format 6 n x m auf ein zweites Format (0 o x p, wobei p = 1 und o ≥ 1, ausgebildet ist.

### Bezugszeichenliste

- 1: Verarbeitungsvorrichtung
- 2: Objekt
- 3: Kontrollierte Umgebung
- 4: Passage
- 5: Gebinde
- 6: erstes Format n x m
- 7: Verpackung
- 8: Umsetzvorrichtung
- 9: Umsetzen
- 10: zweites Format o x p
- 11: Eingangs-Transportvorrichtung
- 12: außen
- 13: Abdeckung
- 14: Objektabdeckung
- 15: Zwischenstation
- 16: Objektaufnahme der Zwischenstation
- 17: Objektaufnahme der Umsetzvorrichtung
- 18: Bearbeitungsstation
- 19: Zwischen-Transportvorrichtung
- 20: Abstandsänderung
- 21: Ausgangs-Umsetzvorrichtung
- 22: Umsetzen der Ausgangs-Umsetzvorrichtung
- 23: Objektaufnahme
- 24: Objektaufnahme-Transportvorrichtung
- 25: Materialfluss der Objekte
- 26: Reinheitszone
- 27: Mover
- 29: Abstand
- 30: Materialfluss der Objektaufnahme
- 31: Greifinstrument
- 32: Gebindeaufnahme
- 33: Arbeitsfläche
- 35: Innenraum
- 36: leerer Platz
- 37: Deckel

## Patentansprüche

1. Verarbeitungsvorrichtung (1) für Objekte (2) in einer kontrollierten Umgebung (3), wobei die kontrollierte Umgebung (3) wenigstens eine Passage (4) zum Einbringen von Objekten (2), die in Gebinden (5) in einem ersten Format (6), das wenigstens eine flächige Anordnung bildet und/oder wenigstens eine Anordnung von n x m Objekten (2), wobei n > 1 und m > 1, umfasst, vorliegen, aufweist, wobei die Gebinde (5) in einer Verpackung (7) bereitgestellt werden, die zumindest teilweise außerhalb der kontrollierten Umgebung (3) verbleibt, **dadurch gekennzeichnet, dass** in der kontrollierten Umgebung (3) eine Umsetzvorrichtung (8) zu einem Umsetzen (9) der Objekte (2) von dem ersten Format (6) n x m auf ein zweites Format (10) o x p , wobei p = 1 und o ≥ 1, ausgebildet ist.

2. Verarbeitungsvorrichtung (1) nach Anspruch 1, **dadurch gekennzeichnet, dass** zwischen der Passage (4) und der Umsetzvorrichtung (8) eine Eingangs-Transportvorrichtung (11) ausgebildet ist, die zum Transport zumindest der Objekte (2), insbesondere der Gebinde (5), im ersten Format (6) eingerichtet ist.

3. Verarbeitungsvorrichtung (1) nach einem der vorangehenden Ansprüche, **dadurch gekennzeichnet, dass** die Verpackung (7) oder ein Teil der Verpackung (7) zu einem Abdichten der Passage (4) eingerichtet ist.

4. Verarbeitungsvorrichtung (1) nach einem der vorangehenden Ansprüche, **dadurch gekennzeichnet, dass** eine Zwischenstation (15) eingerichtet ist, auf der die Objekte (2) im ersten Format (6) verarbeitet werden, insbesondere abgesetzt werden.

5. Verarbeitungsvorrichtung (1) nach einem der vorangehenden Ansprüche, **dadurch gekennzeichnet, dass** die Zwischenstation (15) beweglich, insbesondere verfahrbar und/oder drehbar ist, und/oder dass die Zwischenstation (15) die Objekte (2) für die Umsetzvorrichtung (8) präsentiert.

6. Verarbeitungsvorrichtung (1) nach einem der vorangehenden Ansprüche, **dadurch gekennzeichnet, dass** zumindest ein Mover (27) die Zwischenstation (15) und/oder die Zwischen-Transportvorrichtung (19), so bewegt, insbesondere die zu greifenden Objekte (2) und/oder Absetzpositionen so positioniert, dass die Umsetzvorrichtung (8) bei einer Greifbewegung und/oder Absetzbewegung die Objekte (2) an einem selben Ort wie bei einer vorherigen Greifbewegung und/oder Absetzbewegung greift und/oder absetzt.

7. Verarbeitungsvorrichtung (1) nach einem der vorangehenden Ansprüche, **dadurch gekennzeichnet, dass** die Verarbeitungsvorrichtung (1) wenigstens eine Bearbeitungsstation (18) aufweist, wobei der Bearbeitungsstation (18) vor- und/oder nachgeordnet wenigstens eine Zwischen-Transportvorrichtung (19) eingerichtet ist, insbesondere wobei die Zwischen-Transportvorrichtung (19) zu einem Transport der Objekte (2) im zweiten Format (10) eingerichtet ist, insbesondere wobei eine Dimension o in Transportrichtung der Zwischen-Transportvorrichtung (19) ausgerichtet ist.

8. Verarbeitungsvorrichtung (1) nach einem der vorangehenden Ansprüche, **dadurch gekennzeichnet, dass** eine Zwischen-Umsetzvorrichtung in einer selben Zone wie die Umsetzvorrichtung (8) oder in einer selben Zone wie eine Ausgangs-Umsetzvorrichtung (21) angeordnet ist.

9. Verarbeitungsvorrichtung (1) nach einem der vorangehenden Ansprüche, **dadurch gekennzeichnet, dass** die Verarbeitungsvorrichtung (1) die oder eine Ausgangs-Umsetzvorrichtung (21) aufweist, die die Objekte (2) vom Format o x p oder o x p' in ein Format n' x m' umsetzt, insbesondere in eine Objektaufnahme (23) oder die oder eine Verpackung (7), wobei n' > 1 und m' > 1 ist, insbesondere wobei n`=n und/oder m`=m.

10. Verarbeitungsvorrichtung (1) nach einem der vorangehenden Ansprüche, **dadurch gekennzeichnet, dass** eine Objektaufnahme-Transportvorrichtung (24) separat von der Zwischen-Transportvorrichtung (19) und/oder der Umsetzvorrichtung (8) ausgebildet ist, insbesondere wobei die Objektaufnahme-Transportvorrichtung (24) von einem Materialfluss (25) der Objekte (2) abzweigt und/oder in den oder einen Materialfluss (25) der Objekte (2) mündet.

11. Verarbeitungsvorrichtung (1) nach einem der vorangehenden Ansprüche, **dadurch gekennzeichnet, dass** die Objektaufnahme-Transportvorrichtung (24) zumindest teilweise außerhalb der kontrollierten Umgebung (3) ausgebildet ist.

12. Verarbeitungsvorrichtung (1) nach einem der vorangehenden Ansprüche, dass die Ausgangs-Umsetzvorrichtung (21) und die Umsetzvorrichtung (8) in unterschiedlichen Reinheitszonen angeordnet sind.

13. Verarbeitungsvorrichtung (1) nach einem der vorangehenden Ansprüche, **dadurch gekennzeichnet, dass** die Bearbeitungsstation (18) zu einem gleichzeitigen Verarbeiten, insbesondere Befüllen und/oder Verschließen, von o Objekten (2) und/oder einem Bruchteil b von o Objekten (2) und/oder einem Vielfachen von o Objekten (2) eingerichtet ist, vorzugsweise wobei b ein Teiler von o ist und/oder wobei b= ½*o, 1/3*o, ¼*o ist.

14. Verarbeitungsvorrichtung (1) nach einem der vorangehenden Ansprüche, **dadurch gekennzeichnet, dass** die Umsetzvorrichtung (8) höchstens zwei Bewegungsachsen, insbesondere nur eine Bewegungsachse hat, insbesondere wobei eine oder die Eingangs-Transportvorrichtung (11) und/oder eine oder die Zwischenstation (15) zu einer Positionierung der Objekte (2) relativ zu der Umsetzvorrichtung (8) eingerichtet ist.

15. Verfahren zur Verarbeitung von Objekten (2) in einer kontrollierten Umgebung (3), insbesondere wobei die Objekte (2) als ein Gebinde (5) vorliegen, wobei die Objekte (2) in einer Verpackung bereitgestellt werden, wobei die Objekte (2) in einem ersten Format, das wenigstens eine flächige Anordnung bildet und/oder eine Anordnung von n x m Objekten (2), wobei n > 1 und m > 1, umfasst, durch eine Passage (4) der kontrollierten Umgebung (3) zugeführt werden und in der kontrollierten Umgebung (3) durch eine Umsetzvorrichtung (8) in ein zweites Format (10) o x p, wobei p = 1, o > 1, umgesetzt werden, wobei die Verpackung (7) zumindest teilweise außerhalb der kontrollierten Umgebung (3) verbleibt.

16. Verfahren nach einem der vorangehenden Ansprüche, **dadurch gekennzeichnet, dass** eine Eingangs-Transportvorrichtung (11), die zwischen der Passage (4) und der Umsetzvorrichtung (8) ausgebildet ist, ein Gebinde (5) transportiert.

17. Verfahren nach einem der vorangehenden Ansprüche, **dadurch gekennzeichnet, dass** die Eingangs-Transportvorrichtung (11) die Objekte (2) in dem Format auf einer Zwischenstation (15) absetzt und/oder dass die oder eine Zwischenstation (15) die Objekte (2) der Umsetzvorrichtung (8) präsentiert.

18. Verfahren nach einem der vorangehenden Ansprüche, **dadurch gekennzeichnet, dass** die Objekte (2) nach der Umsetzvorrichtung (8) im zweiten Format (10) transportiert werden.

19. Verfahren nach einem der vorangehenden Ansprüche, **dadurch gekennzeichnet, dass** nach der Umsetzung ein Abstand (29) der Objekte (2) zueinander verändert wird.

20. Verfahren nach einem der vorangehenden Ansprüche **dadurch gekennzeichnet, dass** eine Objektaufnahme (23) in die kontrollierte Umgebung (3) eingebracht wird und/oder dass die oder eine Objektaufnahme (23) nach einer Entnahme der Objekte (2) aus der Objektaufnahme (23) durch die Passage (4) oder eine Schleuse nach außerhalb der kontrollierten Umgebung (3) geschleust wird.

21. Verfahren nach einem der vorangehenden Ansprüche **dadurch gekennzeichnet, dass** die Objektaufnahme (23) zumindest teilweise außerhalb der kontrollierten Umgebung (3) transportiert wird, insbesondere wobei die Objektaufnahme (23) mit den verarbeiteten Objekten (2) bestückt wird und/oder wobei ein Materialfluss (30) der Objektaufnahmen (23) von einem Materialfluss (25) der Objekte (2) abzweigt und/oder in einen Materialfluss (25) der Objekte (2) mündet.

22. Verfahren nach dem vorangehenden Anspruch, **dadurch gekennzeichnet, dass** die umgesetzten Objekte (2) befüllt und/oder verschlossen werden.

23. Verfahren nach einem der vorangehenden Ansprüche, **dadurch gekennzeichnet, dass** gleichzeitig o Objekte (2) und/oder ein Bruchteil b von o Objekten (2) und/oder ein Vielfaches von o Objekten (2), verarbeitet, insbesondere befüllt und/oder verschlossen, werden, vorzugsweise wobei b ein Teiler von o ist und/oder wobei b= ½*o, 1/3*o, ¼*o ist.

24. Verfahren zur Verarbeitung von Objekten (2) in einer kontrollierten Umgebung (3), insbesondere nach einem der vorangehenden Ansprüche, wobei eine Eingangs-Transportvorrichtung (11) und/oder eine Umsetzvorrichtung (8) und/oder eine Zwischen-Umsetzvorrichtung und/oder eine Ausgangs-Umsetzvorrichtung (21) die Objekte (2) mit einem Greifinstrument greift und/oder umsetzt, **dadurch gekennzeichnet, dass** das Greifinstrument (31) zu einer Aufnahme und/oder einem Absetzen so geführt wird, dass ein potenzieller Transport von Verschmutzungen, vom Greifinstrument (31), insbesondere in Bezug auf eine Luftströmung, auf die Objekte (2) geringgehalten wird.

25. Verfahren nach einem der vorangehenden Ansprüche, **dadurch gekennzeichnet, dass** die zu entnehmenden Objekte (2) so ausgewählt werden, dass eine Wegstrecke und/oder ein Überschattungsbereich, die das Greifinstrument (31) über dem Gebinde und/oder der Zwischenstation (15) und/oder der Zwischen-Transportvorrichtung (19) zurücklegt, minimiert und/oder vermieden wird.
